# EUROPEAN PATENT APPLICATION

(11) **EP 3 187 188 A1**
(43) Date of publication of application: **05.07.2017**
(21) Application number: 15835178.3
(22) Date of filing: 30.07.2015
(51) Int. Cl.: A61K 36/185, A61K 8/97, A61K 8/19, A61N 1/06

(54) **METHOD AND APPARATUS FOR DETOXIFYING SCALP USING HENNA**

(30) Priority: 25.08.2014 KR 20140110931
(71) Applicant: EDP Inc, Daejeon 34141 (KR)
(72) Inventor: PARK, Duheon, Daejeon 34152 (KR)
(74) Representative: Carvajal y Urquijo, Isabel
(86) International application number: PCT/KR2015/007973
(87) International publication number: WO 2016/032136

(57) **Abstract**

The present invention is characterized to include a first washing step of removing impurities from scalp and hair, an applying step of applying a mixture of henna and 15 □ to 25 □ water in a ratio of 1:1 to 1:5 to the scalp and the hair and aging for one to two hours with the scalp and the hair covered with an air blocking cover, a second washing step of washing the scalp and the hair to remove the mixture, a drying step of thermally drying the scalp and the hair by a drier providing air at 60 □ to 100 □, and a stimulating step of providing any one or more of an electrical stimulus and a physical stimulus to the scalp for 10 to 30 minutes by a device providing meridian scrapping or a higher frequency.

## Description

### TECHNICAL FIELD

The present invention relates to a method and device for detoxifying scalp using henna, and more specifically, to a method and device for detoxifying scalp using henna, which activate hypodermal cells and increase blood flows in the scalp and prompt scalp waste.

### DISCUSSION OF RELATED ART

Generally, chemically synthesized medicines are used for hair and scalp care. However, the synthetic care medicines may chemically damage hair, and when absorbed in the hair and scalp, may have a negative influence on the human body.

Use of synthetic care medicines may cause inflammation in the sensitive scalp and irritating odors to make people around uncomfortable.

Further, steady use of such synthetic care medicines may inflict chemical damage to the scalp, such as destroying melanin pigment in the hair, turning dark hair into gray, cornifying the scalp, covering the scalp with various toxins and waste, and hardening the scalp.

Further, frequent use of the synthetic care medicines may cause a fever, toxins, and waste to accumulate in the scalp, leading to hair loss.

To address the above issues, Korean Patent Application Publication No. 10-2013-0121057 discloses a hair and scalp care product including the steps of extracting red clay water from the surface of a mixture of red clay and water filtered out by charcoals, the step of obtaining a natural complex extract by fermenting multiple Chinese medicine materials, the step of generating a bio compound using a bio material, and the step of generating a scalp and hair care product including the red clay water, the natural complex extract, and the bio compound.

However, the conventional art requires the process of treating charcoals and red clay multiple times, which is bothering and difficult to treat.

Further, it requires additional hair care and scalp care agents to nourish damaged hair and get rid of waste left on the scalp.

### SUMMARY

To address the above problems, an object of the present invention is to provide a scalp detoxifying method that protects hair by the natural components of henna and provides vegetable proteins to the hair.

Another object of the present invention is to provide a scalp detoxifying method that enhances the scalp condition using henna, prompts hair growth, thickens hair, and retards the hair turning gray.

Still another object of the present invention is to provide a scalp detoxifying method that removes various toxins and waste covering the scalp.

Yet still another object of the present invention is to provide a scalp detoxifying method that may get rid of waste left in scalp pores.

Yet still another object of the present invention is to provide a scalp detoxifying device that provides any one or more of electrical and physical stimuli to the scalp, hair threads, or hair roots to help the scalp and hair threads and roots to absorb active ingredients of henna and to flush out waste.

To achieve the above objects, according to the present invention, a method for detoxifying scalp using henna includes a first washing step of removing impurities from scalp and hair, an applying step of applying a mixture of henna and 15 □ to 25 □ water in a ratio of 1:1 to 1:5 to the scalp and the hair and aging for one to two hours with the scalp and the hair covered with an air blocking cover, a second washing step of washing the scalp and the hair to remove the mixture, a drying step of thermally drying the scalp and the hair by a drier providing air at 60 □ to 100 □, and a stimulating step of providing any one or more of an electrical stimulus and a physical stimulus to the scalp for 10 to 30 minutes by a device providing meridian scrapping or a higher frequency.

The method further includes a meridian scrapping step between the first washing step and the applying step, the meridian scrapping step providing meridian scrapping to the scalp using a meridian scrapping tool including tourmaline to open scalp pores.

In the applying step, bentonite is mixed with the mixture, wherein the henna and the bentonite are in a ratio of 1:0.03 to 1:1, and wherein an aging time is shortened to 30 minutes to one hour.

In the applying step, an 0.1g to 10g oil per 100g of the henna is added to the mixture.

In the stimulating step, a meridian scrapping tool is further used which includes tourmaline to provide the physical stimulus to help blood circulation in the scalp and open the scalp pores.

In the stimulating step, a high-frequency stimulus with a frequency of 20kHz to 30kHz and a voltage of 2kV to 3.5kV is provided to the scalp by a scalp detoxifying device.

According to the present invention, a device for detoxifying scalp using henna includes a body having a handle formed at a lower portion thereof for holding with a hand, a meridian scrapping tool detachably provided to a front end of the body and including tourmaline to provide a physical stimulus to the scalp before and after applying henna to open scalp pores, an oscillator converting input power into a predetermined voltage and frequency in the body, a high-frequency lamp projecting frontward from the front end of the body and generating a high frequency by discharging power with the predetermined voltage and frequency to provide an electrical stimulus to the scalp dried after washing out the henna, and an operation controller controlling an operation of the oscillator.

The operation controller controls the oscillator to generate a high frequency with a frequency of 20kHz to 30kHz and a voltage of 2kV to 3.5kV.

As described above, according to the present invention, the scalp detoxifying method may protect hair with the natural elements of henna and provide vegetable proteins to the hair.

Further, according to the present invention, the scalp detoxifying method may enhance scalp health with henna, prompt hair growth, thicken hair, and retard the hair turning gray.

Further, according to the present invention, the scalp detoxifying method may get rid of various toxins and waste covering the scalp.

Further, according to the present invention, the scalp detoxifying method may remove waste remaining in the scalp pores.

Further, according to the present invention, the scalp detoxifying device may provide any one or more of electrical and physical stimuli to the scalp and hair threads or roots to help the scalp and hair threads and roots to absorb active ingredients of henna while flushing out waste.

### BRIEF DESCRIPTION OF THE DRAWINGS

Fig. 1 is a flowchart schematically illustrating a scalp detoxifying method according to the present invention.
Fig. 2 is a view schematically illustrating a configuration of a scalp detoxifying device according to the present invention.
Fig. 3 is a view schematically illustrating a configuration of a scalp detoxifying device according to an embodiment of the present invention.
Fig. 4 is a view schematically illustrating a configuration of a scalp detoxifying device according to an embodiment of the present invention.
Fig. 5 is a view illustrating before and after applying a scalp detoxifying method to scalp and hair according to the present invention.
Fig. 6 is a view illustrating before and after applying a scalp detoxifying method to scalp and hair according to the present invention.

### DETAILED DESCRIPTION OF EMBODIMENTS

Specific features and advantages of the present invention are described below in detail with reference to the accompanying drawings. When determined to make the gist of the present invention unclear, the detailed description of functions and configurations of the present invention is omitted.

The present invention relates to a method and device for detoxifying scalp using henna, and more specifically, to a method and device for detoxifying scalp using henna, which activate hypodermal cells and increase blood flows in the scalp and prompt scalp waste.

Hereinafter, preferred embodiments of the present invention are described in detail with reference to the accompanying drawings.

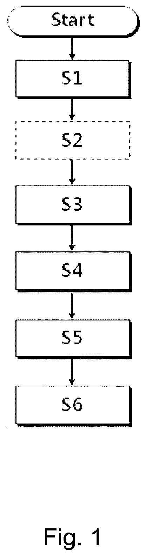

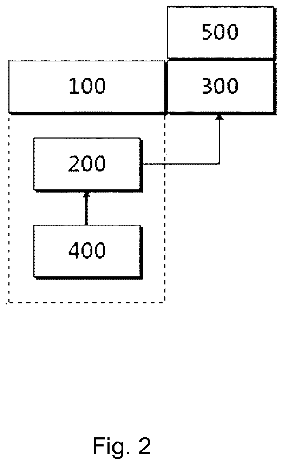

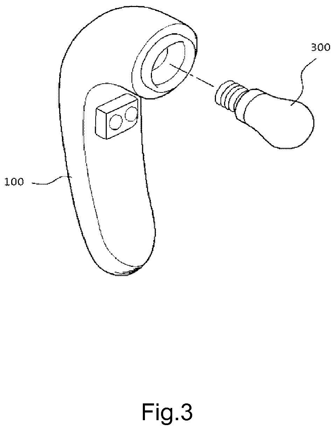

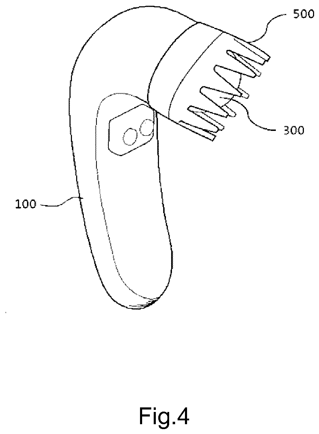

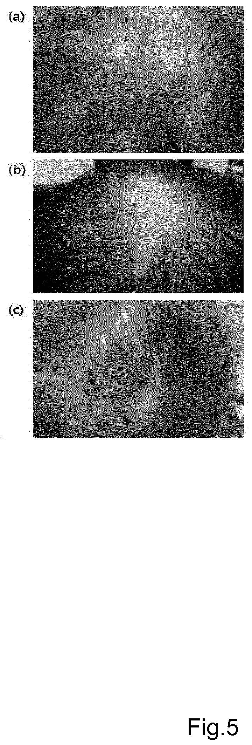

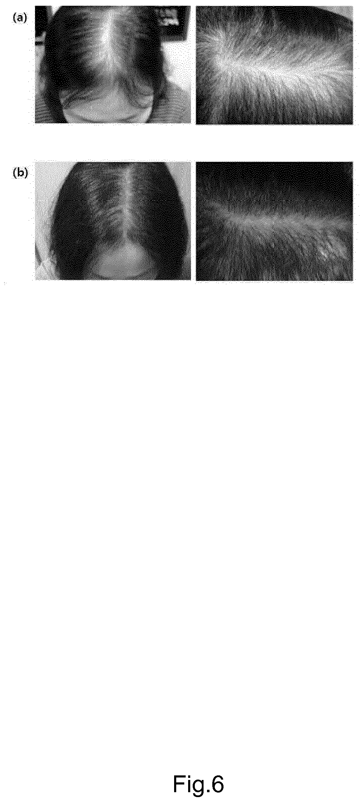

Fig. 1 is a flowchart schematically illustrating a scalp detoxifying method according to the present invention. Fig. 2 is a view schematically illustrating a configuration of a scalp detoxifying device according to the present invention. Fig. 3 is a view schematically illustrating a configuration of a scalp detoxifying device according to an embodiment of the present invention. Fig. 4 is a view schematically illustrating a configuration of a scalp detoxifying device according to an embodiment of the present invention. Fig. 5 is a view illustrating before and after applying a scalp detoxifying method to scalp and hair according to the present invention. Fig. 6 is a view illustrating before and after applying a scalp detoxifying method to scalp and hair according to the present invention.

As shown in Fig. 1, the method includes a first washing step S1 in which waste is removed from scalp and hair, an applying step S3 in which a mixture including henna and 15 □ to 25 □ water mixed in a ratio of 1:1 to 1:5 is applied to scalp and hair and is aged for one to two hours with the scalp and hair covered with an air blocking cover, a second washing step S4 in which the scalp and hair are washed to remove the mixture, a drying step S5 in which the scalp and hair are thermally dried by a dryer providing hot air at 60 □ to 100 □, and a stimulating step S6 in which any one or more of an electrical stimulus and a physical stimulus are provided to the scalp for 10 minutes to 30 minutes by a device for applying a high frequency wave or meridian scrapping.

The first washing step S1 is a step for ridding the hair and scalp of waste and may use a cleanser to remove the fat-soluble waste particularly remaining on the scalp and hair.

If the scalp and hair are washed to remove waste with tepid or warm water, the scalp pores may be expanded to allow the henna mixture to be better absorbed to the scalp and hair in the applying stem S3 that is described below.

The applying step S3 is a step in which the mixture of henna and 15 □ to 25 □ water in a ratio of 1:1 to 1:5 is applied to the scalp and hair and is aged for one to two hours with the scalp and hair covered with the air blocking cover.

Henna has been used as a natural dye and is as safe as it is also used as a medicine for preventing headache, burns, ulcer, and fevers thanks to fever-reducing, sterilizing, and anti-biotic elements contained therein.

Henna also contains a vegetable protein called lawsonia that sticks to the cuticles of hair and scalp to protect and nourish the scalp and hair and drives waste out of the scalp and hair to keep them healthy.

If lawsonia is absorbed to the hair, the overall hair has such an effect as if it is coated, and the hair thickens and becomes glossy.

Powdered henna is difficult to apply to the scalp and hair as it is. Thus, it may be preferable to mix the henna and water in a ratio of 1:1 to 1:5 to obtain a concentration proper to let it easily applied to the hair.

The henna mixture, after applied to the scalp and hair, is aged for one to two hours with the scalp and hair covered with the air blocking cover, such as a plastic wrap or film preventing air influx, so that active elements of henna may be sufficiently absorbed to the scalp and hair.

The scalp detoxifying method according to the present invention may further include a meridian scrapping step S2 between the first washing step S1 and the applying step S3 to provide meridian scrapping to the scalp using a meridian scrapping tool including tourmaline to open the scalp pores.

Meridian scrapping is a therapy using a meridian scrapping tool or bare hands to scrap or tap a portion of a human body or acupuncture points. The meridian scrapping tool (refer to 500 of Fig. 4) is a tool shaped as a horn or comb to facilitate to scrap or tap the human body.

The meridian scrapping tool including tourmaline, when the scalp is stimulated, generates a weak electric current of 0.05mA to 0.06mA closest to the human current in the scalp, and smoothens blood circulation in the scalp and expands scalp pores to allow for better absorption to the scalp and hair roots of the active elements of henna applied to the scalp in the applying step S3.

Tourmaline may bond with water to instantly generate anions and may prompt diabolism of scalp cells to drive the toxins, heavy metals, and waste accumulated in the scalp or hair roots through urine or skin to the outside.

The shape and combined configuration of the meridian scrapping tool is described below in greater detail with reference to Fig. 4.

As shown in Figs. 2 and 3, the scalp detoxifying agent according to the present invention includes a body 100 having a handle formed at its lower part to hold with a hand, a meridian scrapping tool 500 including tourmaline and detachably provided to a front portion of the body to provide a physical stimulus to the scalp before and after applying henna to open scalp pores, an oscillator 200 converting input power into a predetermined voltage and frequency in the body, a high-frequency lamp 300 projecting to the front from a front portion of the body and generating a high frequency by discharging the power with the predetermined voltage and frequency to provide an electrical stimulus to the scalp that has been washed and then dried, and an operation controller 400 controlling the operation of the oscillator 200.

The body 100 has the handle formed at a lower portion thereof for holding with a hand and may be included inside the oscillator 200 and the operation controller 400 described below.

Further, the body 100 may include a status display window to display the status of the scalp detoxifying device according to the present invention and may further include control buttons or switches to turn on/off the power or input control commands to the oscillator 200 and the operation controller 400.

Further, the body 100 may include any one or more of an internal battery and a power input unit to supply power that may be required for each component in the scalp detoxifying device according to the present invention.

The meridian scrapping tool 500 may contain tourmaline, be detachably provided to a front end of the body, and may provide a physical stimulus to the scalp before and after applying the henna to open scalp pores.

The tourmaline powder-added meridian scrapping tool generates a weak current of 0.05mA to 0.06mA which is closest to the human current in the scalp when providing the physical stimulus to cause a friction. Thus, the blood circulation in the scalp is enhanced, and scalp pores are opened.

The oscillator 200 is a component to convert input power into a predetermined voltage and frequency, and it may perform such conversion so that energy, which is externally discharged through the high-frequency lamp 300 described below, may be safe to the human body.

Preferably, it may be safe to convert it to have a frequency of 20kHz to 30kHz and a voltage of 2kV to 3.5kV that are harmless to the human body.

The high-frequency lamp 300 is formed to project to the front from the front end of the body so that it can reach the scalp. The high-frequency lamp 300 may provide a high-frequency to the scalp by discharging the power with the predetermined voltage and frequency converted by the oscillator 200 to provide an electrical stimulus to the scalp that has been dried after washing out the henna.

Referring to Fig. 3, the high-frequency lamp 300 may be fittingly coupled to the front end of the body 100 and may receive the power converted through the oscillator 200 includable in the body 100 and cause an electric discharge to the user's scalp.

The operation controller 400 controls the operation of the oscillator 200 so that the oscillator generates a high frequency of 20kHz to 30kHz and a voltage of 2kV to 3.5kV.

As shown in Fig. 4, the meridian scrapping tool 500 including tourmaline may be coupled to the front end of the body 100 of the scalp detoxifying device according to the present invention to provide meridian scrapping which helps blood circulation in the scalp and opens scalp pores.

The meridian scrapping tool 500 may couple or decouple from the front end of the body 100. To provide a physical stimulus to the scalp, the meridian scrapping tool 500 may be coupled to the front end of the body 100 to be used for the scalp. To provide an electrical stimulus, the meridian scrapping tool 500 may be decoupled from the body 100 so that the high-frequency lamp 300 may contact the scalp and generate a high frequency.

Meridian scrapping is a therapy to treat a human body by scrapping or tapping using bare hands or a meridian scrapping tool. The meridian scrapping tool 500 may be a tool shaped as a comb to facilitate to provide such a therapy as scrapping or tapping the human body.

The meridian scrapping tool 500 according to the present invention may be shaped as a crown having an empty middle region for easier coupling to the front end of the body 100, and it may be separated by forcedly pulling in an opposite direction of the direction of coupling. Thus, coupling and decoupling are easy.

Further, the meridian scrapping tool 500 may be coupled to the front end of the body 100 in a form surrounding the high-frequency lamp 300 so that a high frequency and meridian scrapping may be simultaneously provided to the scalp or human body.

Further, the meridian scrapping tool 500 contains a tourmaline powder and when meridian scrapping is provided to the scalp a weak electric current of 0.05mA to 0.06mA which is closest to the human electric current is generated by a friction, and this may enhance blood circulation and expand scalp pores.

Tourmaline may bond with water to instantly generate anions, to prompt diabolism, and to discharge toxins, heavy metals, and waste accumulated on the skin through sweat, urine, and skin to the outside of the body.

Further, the high-frequency lamp 300 may radiate a blue wavelength of light to kill dermodex mites, preventing inflammation.

Addition of the infrared (IR) lamp may help absorption of the henna mixture to the scalp and hair threads and roots and enhance the blood circulation in the scalp to assist in discharge of waste accumulated on the scalp.

Fig. 5 illustrates, in the up-to-down order, a status (a) before the scalp detoxifying method according to the present invention is applied to the hair, a status (b) after the scalp detoxifying method has been applied as many as a preferable number of times for applying the scalp detoxifying method for 20 days, and a status (c) after the scalp detoxifying method has been applied as many as a preferable number of times for applying the scalp detoxifying method for 30 days.

Referring to Fig. 5, before the scalp detoxifying method is applied (status (a)), the hair had fewer and thinner hair threads and much waste accumulation covering the scalp. 20 days after the scalp detoxifying method has applied, the waste inside the scalp or covering the scalp is out of the scalp, and more hair threads grow. In 30 days, the hair threads thicken, and the hair threads and roots turn healthy and darker.

Fig. 6 illustrates a status (a) before the scalp detoxifying method according to the present invention applies to the scalp and hair and a status (b) after the scalp detoxifying method has applied to the scalp and hair ten times.

Before the scalp detoxifying method applies, the hair is thin and light in color, but after the scalp detoxifying method has applied ten times, noticeably many new hair threads have grown, and the hair has thickened and darkened, and the blood circulation in the scalp is more smoothly done.

The scalp detoxifying method according to the present invention is preferably provided every day for a first week, and ten times after it has applied, it is preferably provided three times per week.

If the scalp detoxifying method is provided to the scalp and hair for three weeks, the hair may turn thicker, darker, and glossier, and mitigate hair loss while prompting hair growth.

Further, the scalp detoxifying device according to the present invention may include a frequency detector that may detect a difference between an output frequency radiated to the scalp through the high-frequency lamp 300 and an input frequency reflected from the scalp to determine whether the skin type of scalp is oily, normal, or dry.

The scalp impendence varies depending on the moisture of the scalp, and a difference between the output frequency and the input frequency is rendered to occur by the scalp impedance. Thus, the user's scalp type may be determined.

The frequency detector determines that the type of scalp is oily in case the input frequency is smaller than the output frequency within 5kHz to 10kHz, normal in case the input frequency is smaller than the output frequency within 3kHz to 5kHz, and dry in case the input frequency is smaller than the output frequency within less than 3kHz.

If the user's scalp type is determined, a voltage and frequency stimulus suited for each type may be provided. If a user-tailored high frequency is provided to the dermis of the scalp, blood circulation smooths and cells are activated, leading to prompted waste discharge.

If the scalp is determined to be of oily type by the frequency detector, a 20kHz frequency is gradually increased at a ratio of 20% to 30% at 30sec intervals, while a 2kV voltage is increased by 300V at 30sec intervals.

Since the oily scalp indicates that the scalp has excessive moisture, radiating a high frequency at an early stage may damage the scalp. Accordingly, starting with a lower wavelength frequency and a lower voltage, the frequency radiated is gradually increased so that the high frequency slowly penetrates into the dermis of the scalp.

Further, the frequency and the voltage are rendered not to exceed 30kH and 3kV, respectively, to prevent damage to the scalp.

In case the scalp is of normal type, the operation controller preferably increases the 25kHz frequency at a ratio of 10% to 20% at 30sec intervals while increasing the 2.5kV voltage by 300V at 30sec intervals.

Since the normal-type scalp contains a proper amount of moisture, it may be efficient to start with a higher frequency and voltage than those of the oily-type scalp.

Further, because of starting with a higher frequency than that of the oily-type scalp, the frequency increase ratio is set to be lower than that of the oily-type scalp, and the frequency and voltage are set not to exceed 30kHz and 3.5kV, respectively, to prevent damage to the scalp.

Further, in case the scalp is of dry type, it indicates that the scalp contains insufficient moisture, and thus, long-term provision of a high frequency and voltage may easily do damage to the scalp. Thus, it may be preferable to first provide a high frequency and high voltage and gradually reduce the frequency and voltage.

Further, it may be preferable to maintain the frequency and the voltage not to be less than 20kHz and 2kV, respectively.

As described above, according to the present invention, the scalp detoxifying method may protect hair with the natural elements of henna, provide vegetable proteins to the hair, enhance scalp health with henna, prompt hair growth, thicken hair, and retard the hair turning gray, get rid of various toxins and waste covering the scalp, and remove waste remaining in the scalp pores.

Further, according to the present invention, the scalp detoxifying device may provide any one or more of electrical and physical stimuli to the scalp and hair threads or roots to help the scalp and hair threads and roots to absorb active ingredients of henna while flushing out waste.

While the present invention has been shown and described with reference to exemplary embodiments thereof, it will be apparent to those of ordinary skill in the art that various changes in form and detail may be made thereto without departing from the spirit and scope of the present invention as defined by the following claims.

## Claims

1. A method for detoxifying scalp using henna, comprising:
a first washing step of removing impurities from scalp and hair;
an applying step of applying a mixture of henna and 15 □ to 25 □ water in a ratio of 1:1 to 1:5 to the scalp and the hair and aging for one to two hours with the scalp and the hair covered with an air blocking cover;
a second washing step of washing the scalp and the hair to remove the mixture;
a drying step of thermally drying the scalp and the hair by a drier providing air at 60 □ to 100 □; and
a stimulating step of providing any one or more of an electrical stimulus and a physical stimulus to the scalp for 10 to 30 minutes by a device providing meridian scrapping or a higher frequency.

2. The method of claim 1, further comprising a meridian scrapping step between the first washing step and the applying step, the meridian scrapping step providing meridian scrapping to the scalp using a meridian scrapping tool including tourmaline to open scalp pores.

3. The method of claim 1, wherein in the applying step, bentonite is mixed with the mixture, wherein the henna and the bentonite are in a ratio of 1:0.03 to 1:1, and wherein an aging time is shortened to 30 minutes to one hour.

4. The method of claim 3, wherein in the applying step, an 0.1g to 10g oil per 100g of the henna is added to the mixture.

5. The method of claim 1, wherein in the stimulating step, a meridian scrapping tool is further used which includes tourmaline to provide the physical stimulus to help blood circulation in the scalp and open the scalp pores.

6. The method of claim 1, wherein in the stimulating step, a high-frequency stimulus with a frequency of 20kHz to 30kHz and a voltage of 2kV to 3.5kV is provided to the scalp by a scalp detoxifying device.

7. A device for detoxifying scalp using henna, comprising:
a body having a handle formed at a lower portion thereof for holding with a hand;
a meridian scrapping tool detachably provided to a front end of the body and including tourmaline to provide a physical stimulus to the scalp before and after applying henna to open scalp pores;
an oscillator converting input power into a predetermined voltage and frequency in the body;
a high-frequency lamp projecting frontward from the front end of the body and generating a high frequency by discharging power with the predetermined voltage and frequency to provide an electrical stimulus to the scalp dried after washing out the henna; and
an operation controller controlling an operation of the oscillator.

8. The device of claim 7, wherein the operation controller controls the oscillator to generate a high frequency with a frequency of 20kHz to 30kHz and a voltage of 2kV to 3.5kV.
